# EUROPÄISCHE PATENTSCHRIFT

(11) **EP 2 774 530 B1**
(45) Veröffentlichungstag und Bekanntmachung des Hinweises auf die Patenterteilung: **10.07.2019**
(21) Anmeldenummer: 13157616.7
(22) Anmeldetag: 04.03.2013
(51) Int. Cl.: A61B 5/00

(54) **Verfahren zur photoakustischen Tomographie**
Method for photoacoustic tomography
Procédé de tomographie photoacoustique

(43) Veröffentlichungstag der Anmeldung: 10.09.2014
(73) Patentinhaber: Medizinisches Laserzentrum Lübeck GmbH, 23562 Lübeck (DE)
(72) Erfinder: Horstmann, Jens, 24106 Kiel (DE); Brinkmann, Ralf, 23562 Lübeck (DE)
(74) Vertreter: RCD Patent Giesen, Schmelcher & Griebel Patentwanwälte PartG mbB

(56) Entgegenhaltungen:
- ZHENHENG LIN ET AL: "Study of photoacoustic imaging based on all-optical detection", PROCEEDINGS OF SPIE, Bd. 7160, 3. Dezember 2008 (2008-12-03), Seiten 71602K-71602K-8, XP055072210, ISSN: 0277-786X, DOI: 10.1117/12.807018
- BJÖRN KEMPER ET AL: "Endoscopic Double-Pulse Electronic-Speckle-Pattern Interferometer for Technical and Medical Intracavity Inspection", APPLIED OPTICS, Bd. 39, Nr. 22, 1. August 2000 (2000-08-01), Seite 3899, XP055072671, ISSN: 0003-6935, DOI: 10.1364/AO.39.003899
- CRISTINA TRILLO ET AL: "Numerical reconstruction of acoustic bulk waves in aluminium from TV holography surface displacement measurements", PROCEEDINGS OF SPIE, Bd. 7098, 4. Juni 2008 (2008-06-04), Seiten 70980G-70980G-9, XP055072227, ISSN: 0277-786X, DOI: 10.1117/12.802984

## Beschreibung

Die Erfindung betrifft ein Verfahren zur photoakustischen Tomographie von Probenvolumina, die in ihrem Innern spektral adressierbare Absorptionskontraste für elektromagnetische Strahlung für wenigstens eine Zielstruktur aufweisen. Die Erfindung betrifft auch eine Vorrichtung zur photoakustischen Lokalisierung von physiologischen Strukturen im Innern eines biologischen Gewebes.

Bei der Photoakustischen Tomographie (PAT) wird Anregungslicht aus einem vorbestimmten Teilspektrum des elektromagnetischen Spektrums auf ein Probenvolumen eingestrahlt. Das Anregungslicht soll in das Probeninnere eintreten und dabei ggf. auch gestreut, aber nur schwach bis gar nicht vom überwiegenden Teil des Probenmaterials absorbiert werden. Andererseits sollen vorbestimmte Zielstrukturen, die in a priori unbekannter Verteilung im Probeninnern vorliegen, das Anregungslicht unter Erwärmung absorbieren und so eine lokalisierte thermische Expansion des Absorbers und im Grenzbereich auch des umliegenden Probenmaterials bewirken.

Das vorbestimmte Teilspektrum der Anregungsstrahlung richtet sich nach den Materialeigenschaften der Probe und insbesondere auch nach denen der Zielstrukturen, die photoakustisch lokalisiert werden sollen. Der Nutzer wird sich ein geeignetes Teilspektrum für seine konkrete Probe im Einzelfall selbst wählen müssen. Ein Probenvolumen, das im Innern "spektral adressierbare" Absorptionskontraste für eine Zielstruktur aufweist, soll per Definition ein solches sein, für das man ein Teilspektrum des elektromagnetischen Spektrums finden kann, aus dem Licht von der Zielstruktur anders als von der Umgebung der Zielstruktur absorbiert wird.

Die PAT kann prinzipiell auch angewandt werden, um Zielstrukturen zu lokalisieren, die das Licht schlechter als ihre Umgebung absorbieren und dadurch die Druckwellenausbreitung im Material messbar stören, beispielsweise Lufteinschlüsse im Material oder andere Materialdefekte (Defekt-Screening).

Gewöhnlich ist bei der PAT das Absorptionsvermögen für die Anregungsstrahlung in einer Zielstruktur höher als in der Umgebung der Zielstruktur. Der besseren Verständlichkeit halber soll im Weiteren nur diese Situation besprochen werden, wobei die Übertragbarkeit auf das Defekt-Screening als fachmännisch anzusehen ist.

Erfolgt die Einstrahlung des Anregungslichts auf die Probe gepulst, so werden im Innern der Probe zeitlich begrenzte Druckwellen ausgelöst (photoakustischer Effekt). Die durch Absorption im Innern erzeugten und sich hiernach zur Probenoberfläche fortpflanzenden Druckwellen führen zu kleinen und kurzzeitigen Deformationen der Probenoberfläche.

Dies setzt allerdings voraus, dass die Pulsenergie des Anregungslichts einen angemessenen Wert aufweist, der ebenfalls vom Nutzer für seine konkrete Probe selbst zu wählen ist. Ist die Pulsenergie zu gering, dissipiert die eingetragene Energie während der Druckwellenpropagation, so dass praktisch keine Deformationen detektierbar sind. Eine zu hohe Pulsenergie kann die Probe indes irreversibel schädigen.

Liegt die Pulsdauer eines Anregungslichtpulses in der Größenordnung von einigen Nanosekunden, bevorzugt im Bereich der akustischen Einschlusszeit der Absorber, und die Bestrahlung im Bereich von einigen mJ/cm², so hat man mit Deformationsamplituden an der Probenoberfläche in der Größenordnung von einigen Nanometern über eine Zeitspanne von Mikrosekunden zu rechnen.

Die Detektion der Deformationen der Probenoberfläche und die anschließende Auswertung ihres räumlichen und zeitlichen Verhaltens erlauben die Lokalisierung und ggf. sogar die Qualifizierung und Quantifizierung der Zielstrukturen im Innern der Probe.

In der Arbeit von Carp und Venugopalan, "Optoacoustic imaging based on the interferometric measurement of surface displacement", Journal of Biomedical Optics, Vol. 12(6), 2007, S. 064001 ff. wird beispielsweise erläutert, wie man aus den zeit- und ortsaufgelösten Deformationsdaten bei vorbekannter Schallgeschwindigkeit der Probe die Quellenverteilung der Druckwellen voxelweise rekonstruiert (vgl. dort Fig. 3 und Text). Solche auf "back projection" beruhenden Rekonstruktionsverfahren finden breite Anwendung in der Computer-Tomographie (CT) oder Digitalen Tomosynthese (DT). Es handelt sich gemeinhin um rechenintensive Methoden, die erst nach dem Abschluss der vollständigen Messdatenerfassung durchgeführt werden.

Im Weiteren wird davon ausgegangen, dass die Rekonstruktion der Quellenverteilung der optisch angeregten Druckwellen ein an sich gelöstes Problem ist. Die PAT ist im Prinzip durchführbar, sobald Deformationen der Probenoberfläche ausreichend genau und mit ausreichender Zeitauflösung detektiert werden können.

Von Carp und Venugopalan wird beispielsweise eine Messung von mit einem gepulsten Anregungslaserstrahl erzeugten Druckoszillationen beschrieben. Der Messaufbau basiert dort auf einem Mach-Zehnder-Interferometer, das mit einem He-Ne-Laser als Beobachtungslichtquelle arbeitet. Das Beobachtungslaserlicht wird dichroitisch in den Strahlengang des Anregungslasers eingespiegelt und vornehmlich von der Probenoberfläche gestreut. Das Interferometer detektiert Verschiebungen der Probenoberfläche aus der Phasenverschiebung des Beobachtungslaserlichts gegen eine vorgegebene Referenzoszillation, die durch einen ansteuerbaren akustooptischen Modulator eingeführt wird.

Es ist besonders wünschenswert, die Druckwellendetektion kontaktlos allein durch Beleuchtung, Abbildung und zeitliche Beobachtung der Probenoberfläche durchzuführen und auf jegliche Anordnung von Druckaufnehmern auf der Probe zu verzichten. Druckaufnehmer können einerseits die Probe gegen die Anregungsstrahlung abschatten und bedürfen andererseits einer akustischen Impedanzanpassung an das Probenmaterial.

Bei Carp und Venugopalan wird das Beobachtungslaserlicht punktuell appliziert im Unterschied zum flächig applizierten Anregungslicht. Es lassen sich so punktweise Interferenzphasen und damit Oberflächenverschiebungen bestimmen, wobei in jedem Messpunkt eine komplette Zeitreihe von Signalen aufgezeichnet werden kann. Zur Feststellung der räumlichen Verteilung der Oberflächendeformationen der Probe ist es erforderlich, den mit Beobachtungslicht bestrahlten Messpunkt relativ zur Probe zu verschieben, was z.B. hier mit einem senkrecht zur Einstrahlrichtung beweglichen Probenhalter geschieht. Solche Verschiebungen erfordern je ca. 0,5 s Messpause, wozu sich noch 0,3 bis 0,5 s Messzeit pro Messpunkt für 350 bis 750 Messpunkte addieren, bis eine Probenoberfläche von einigen Quadratzentimetern abgerastert worden ist. Die Autoren geben 7 bis 15 Minuten Akquisitionsdauer für alle zur tomographischen Rekonstruktion erforderlichen Daten an.

Für Anwendungen, bei denen die Probe nicht in einer vibrationsgeschützten Halterung fest eingespannt werden kann, ist eine Datenakquisition über etliche Minuten ungünstig. Ist die Probe sogar ein lebendes Gewebe, dann muss mit unvermeidlichen Eigenbewegungen der Probe gerechnet werden, die das Messergebnis stören oder völlig unbrauchbar machen. Außerdem kann eine generelle Erwärmung der Probe durch die wiederholte Einstrahlung von Anregungslicht unterstellt werden, die zur Expansion der Probe während der Messdauer führt.

ZHENHENG LIN ET AL: "Study of photoacoustic imaging based on all-optical detection", PROCEEDINGS OF SPIE, Bd. 7160, 3. Dezember 2008 (2008-12-03), Seiten 71602K-71602K-8, ISSN: 0277-786X, zeigt einen auf ESPI beruhenden Aufbau zur Detektion von transienten Oberflächenverformungen. Das Verfahren beruht auf Einzelpulsen und ist nicht zur Beobachtung von nicht ruhenden Gegenständen geeignet.

Eine Möglichkeit zur Verkürzung der Messdauer kann darin gesehen werden, dass man die ortsabhängige Deformation der Probenoberfläche als Funktion der Zeit an möglichst vielen Orten gleichzeitig detektiert. Die Gesamtheit der Deformationsmesswerte einer Messfläche auf der Probenoberfläche zu einem festen Zeitpunkt soll im Weiteren als Deformationsprofil bezeichnet werden. Eine zeitliche Sequenz von Deformationsprofilen mit einer ausreichend hohen Stützstellendichte bezüglich Zeit und 2D-Ortskoordinaten (der Messfläche) ist ein geeigneter Ausgangsdatensatz für eine tomographische Rekonstruktion. Man kann erwägen, zu seiner Gewinnung auf einen Messaufbau mit einer digitalen Kamera und auf ein holographisches Verfahren aus der Elastometrie zurückgreifen.

Dem Begriff "Doppelpuls Elektronische Speckle-Interferometrie (DP-ESPI)" kommt in der vorliegenden Erfindung große Bedeutung zu. Nachfolgend wird klargestellt, was darunter im Kontext der vorliegenden Anmeldung zu verstehen ist.

Im weitesten Sinne ist die DP-ESPI eine Methode zur Detektion von transienten Vibrationen einer Oberfläche, bei der zwei zeitlich nacheinander durchgeführte Beleuchtungen der Oberfläche mit kohärentem Licht und die Aufzeichnung wenigstens je eines durch die Streuung des Lichts bei einer Beleuchtung erzeugten Specklemusters auf einem zweidimensionalen Lichtdetektorarray (i. F. elektronische Kamera mit lichtsensitiven Pixeln) erfolgen. Bei einem ausreichend kurzen zeitlichen Abstand der Beleuchtungen und bei nur sehr geringen Deformationen sind die einzelnen Speckles der wenigsten zwei Specklebilder nahezu unverändert mit Ausnahme ihrer Phasen, die sehr empfindlich vom Abstand der einzelnen Bereiche der streuenden Oberfläche zur Kamera abhängen. Bei der DP-ESPI wird grundsätzlich die Phasendifferenz der Speckles zwischen den beiden Beleuchtungszeitpunkten bestimmt und hieraus auf die in der Zwischenzeit erfolgte lokale Bewegung der Oberfläche geschlossen.

Eine mögliche Ausführungsform der DP-ESPI ist aus der Arbeit von Pedrini et al., "Double-pulse electronic speckle interferometry", JOURNAL OF MODERN OPTICS, 1993, VOL. 40, No.1, 89-96 bekannt. In dieser Arbeit wird berichtet von der Messung der Deformationen einer Metallplatte, die mit einem Pendelanschlag zu Schwingungen erregt wird. Die Plattenfläche wird dabei mit Beobachtungslaserlicht beleuchtet und auf eine CCD-Kamera abgebildet. Ein kleiner Anteil (10 %) des Beobachtungslichts wird als Referenzstrahl vor der Probe ausgespiegelt. Ein Anteil des übrigen Beobachtungslichts wird von der Probe gestreut und als Probenstrahl durch eine Apertur zur CCD-Kamera geführt. Der Zweck der Apertur liegt darin, die mittlere Größe der im Probenstrahl vorhandenen Speckles auf etwa die vierfache Breite der Kamerapixel zu erhöhen. Zugleich erfolgt der Einfall des Referenzstrahls auf die Kameraebene unter einem Winkel gegenüber dem Probenstrahl, um eine Phasenrampe - und damit auch ein Fringemuster der Interferenzlichtintensität - entlang der Pixelanordnung einzurichten (vgl. dort Fig. 4). Ist die Periodenlänge des Fringemusters kleiner als der mittlere Speckledurchmesser und zugleich wenigstens drei (hier: vier) Kamerapixelabstände groß, dann kann aus zwei in kurzem Abstand nacheinander aufgezeichneten Specklemustern ein Phasenprofil unmittelbar berechnet werden, das die Deformation der Probe zwischen den beiden Beleuchtungspulsen wiedergibt. Ein Deformationsprofil ergibt sich hieraus mittels "phase unwrapping" (vgl. dort Fig. 5, insbesondere Fig. 5 e).

Prinzipiell kann auf Einführung des Interferenzwinkels zwischen Referenzstrahl und Objektstrahl verzichtet werden, wenn der geometrische Weg des Referenzstrahls in Relation zum Objektstrahl definiert im Subwellenlängenbereich verändert werden kann, z.B. durch bewegliche Spiegel. In diesem Fall ist die sequentielle Aufnahme von mindestens drei Aufnahmen mit abweichenden Phasenlagen zur Bestimmung eines Phasenprofils nötig. Bekannt ist dies als ESPI-Variante "Temporal Phase Shifting", die allerdings für zeitkritische Vorgänge aufgrund der zeitlich nicht beliebig schnell ansteuerbaren Spiegel nur bedingt geeignet ist.

Eine hierzu alternative Lösung besteht darin, statt *eines* lichtempfindlichen Sensors mindestens drei zu verwenden. Das Licht, welches durch Interferenz zwischen Objektstrahl und Referenzstrahl die optische Phase beinhaltet, muss nach dem ESPI-Prinzip mit mindestens drei bekannten Phasenlagen zwischen den beiden Strahlen abgebildet werden. Wird mittels mehrerer Strahlteiler das Licht auf mindestens drei Sensoren verteilt, die entsprechend der geforderten Phasenlagen geometrisch feinjustierbar oder bereits entsprechend konstruiert sind, lassen sich die drei Interferenzbilder simultan aufzeichnen. Damit ist das Verfahren ebenso für zeitkritische Vorgänge einsetzbar und prinzipiell gleichwertig dem oben beschriebenen Ansatz unter Verwendung einer Phasenrampe.

Es kann auch eine Farbkamera, mithin ein Sensor mit einer spektral abhängigen Pixelanordnung, verwendet werden. Vor den zueinander benachbarten Pixeln sind hierbei in periodischer Wiederholung verschiedene Farbfilter angeordnet. Zur Beleuchtung der Probe werden zeitgleich mindestens drei verschiedene Wellenlängen genutzt, für die dann jeweils nur ein Teil der Kamerapixel sensitiv ist. Die Modifikation der optischen Weglänge ist etwa über dichroitische Elemente im Strahlengang zu realisieren.

In diesem Sinne sind weitere prinzipiell gleichwertige Modifikationen denkbar. Eine strukturierte Phasenmaske vor dem Sensor kann beispielsweise definiert je Pixel die optische Weglänge modifizieren.

Das Prinzip der Doppelpuls-ESPI wird vielfach zur Detektion der Änderung der Oberflächentopographie eingesetzt, wie es z.B. BJÖRN KEMPER ET AL: "Endoscopic Double-Pulse Electronic-Speckle-Pattern Interferometer for Technical and Medical Intracavity Inspection", APPLIED OPTICS, Bd. 39, Nr. 22, 1. August 2000 (2000-08-01), Seite 3899, ISSN: 0003-6935 zeigen. Dabei erlaubt das Verfahren einen Ersatz für eine taktile Rückmeldung an einen Operateur.

Nach Kenntnis der Erfinder wurde bislang noch nicht versucht, die DP-ESPI in die Photoakustische Tomographie einzubinden. Ein Problem dabei ist darin zu sehen, dass die Anregung von Druckwellen bei der PAT "irgendwann" und "irgendwo" in der Probe erfolgt und somit nicht klar ist, wie man die mit der DP-ESPI ermittelbaren Deformationsprofile in einen für die PAT-Rekonstruktion geeigneten zeitlichen Bezugsrahmen zu setzen hat. Weiterhin ist bei biologischen Geweben die Oberfläche bzgl. der Speckleentstehung nicht klar definiert, da das Beobachtungslicht in die Probe eindringt und somit die Speckle einer Volumenstreuung zuzuordnen sind, und die Speckleverschiebung auf Änderungen in diesem oberflächennahen Volumen beruhen. Dies erschwert die Rücktransformation auf die Lokalisation der Absorber. Überdies muss man die Messfläche aufgrund der a priori unbekannten Laufzeiten der Druckwellen zur Messfläche kontinuierlich, d.h. zumindest mit einer Zeitauflösung der Größenordnung Nanosekunden, über mehrere Mikrosekunden hinweg beobachten. Es ist nicht ohne weiteres ersichtlich, wie man dies mit einer heute verfügbaren elektronischen Kamera erreichen kann.

Die Aufgabe der Erfindung ist es, ein Verfahren zur Photoakustischen Tomographie vorzuschlagen, das eine schnellere Datenerfassung zur Messung der Deformationen der Probenoberfläche als im Stand der Technik ermöglicht.

Die Aufgabe wird gelöst durch ein Verfahren zur Photoakustischen Tomographie einer Probe, wobei an einer Messfläche auf der Probe Deformationen als Funktion von Ort und Zeit gemessen werden, die sich durch Absorption einer gepulsten Anregungsstrahlung an wenigstens einer spektral adressierbaren Zielstruktur im Probeninneren unter Emission thermomechanischer Druckwellen in Richtung der Messfläche ergeben, und wobei die gemessenen Deformationen einem Rekonstruktionsverfahren zur Bestimmung der Lage der Zielstruktur im Probeninneren zugeführt werden, dadurch gekennzeichnet, dass
a. zu einem Anregungspuls ein vorlaufender und ein nachlaufender Beobachtungslaserpuls auf die Messfläche eingestrahlt werden, wobei
b. ein zweidimensionales Deformationsprofil der Messfläche aus den von der Messfläche gestreuten Beobachtungslaserpulsen mittels der Methode der Doppelpuls Elektronischen Speckle-Interferometrie (ESPI) bestimmt wird und
c. der Zeitabstand zwischen dem Anregungspuls und dem nachlaufenden Beobachtungslaserpuls dem zuvor bestimmten Deformationsprofil als Zeitindex zugeordnet wird und
d. eine vorbestimmte Zahl von Wiederholungen der Schritte a bis c unter Variieren wenigstens des Zeitabstandes zwischen Anregungspuls und nachlaufendem Beobachtungslaserpuls erfolgen.

Die Unteransprüche geben vorteilhafte Ausgestaltungen an.

Erfindungsgemäß wird aus zwei nacheinander durchgeführten Beleuchtungen einer Messfläche auf der Probe mit Beobachtungslaserlicht ein Deformationsprofil ermittelt und auf den Zeitabstand eines dritten Lichtpulses, der *zwischen den beiden* Beobachtungslaserpulsen appliziert wird, zum späteren der beiden Beobachtungslaserpulse bezogen.

Dieser dritte Lichtpuls soll in die Probe eintreten und von der Zielstruktur absorbiert werden. Er stellt das Anregungslicht der PAT-Messung dar und stammt aus einem anderen Teilbereich des elektromagnetischen Spektrums als das Beobachtungslaserlicht.

Die Abfolge der drei Lichtpulse wird N-mal wiederholt (wobei N eine vorgebbare natürliche Zahl ist) unter Verändern wenigstens des genannten Zeitabstandes, um eine Sequenz von N Deformationsprofilen zu N verschiedenen Zeitindizes zu erzeugen.

Der in der erzeugten Sequenz den Deformationsprofilen zugeordnete Abstand zweier aufeinanderfolgender Zeitindizes ist in der Regel sehr viel kleiner als die tatsächliche Zeitspanne, die zwischen der messtechnischen Erfassung zweier aufeinanderfolgender Deformationsprofile liegt. Gleichwohl approximiert die durch Wiederholung der Abfolge der drei Lichtpulse gewonnene Sequenz eine "wahre Sequenz von Deformationsprofilen", die infolge nur eines einzigen Anregungspulses auf der Messfläche binnen weniger Mikrosekunden nach dem Anregungspuls auftritt. Die Erfassung der "wahren Sequenz" würde jedoch technisch voraussetzen, über eine elektronische Kamera zu verfügen die mehrere Millionen Bilder pro Sekunde aufzeichnen könnte. Der Stand der Kameratechnik liegt derzeit allerdings drei Größenordnungen darunter. Die erfindungsgemäß erzeugte Sequenz stellt hingegen ein realisierbares Messergebnis und zugleich einen adäquaten Ausgangsdatensatz für eine tomographische Rekonstruktion im Sinne der PAT dar.

Ein wichtiger Vorzug der Erfindung gegenüber dem Stand der Technik ist darin zu sehen, dass die messtechnische Erfassung aller Rohdaten zur nachträglichen Errechnung der Sequenz von Deformationsprofilen ohne weiteres in weniger als zehn Sekunden, vorzugsweise in weniger als einer Sekunde, besonders bevorzugt in weniger als 200 Millisekunden abgeschlossen werden kann.

Die Erfindung wird nachfolgend näher erläutert auch anhand von Figuren. Dabei zeigt:
- Fig. 1: eine Skizze der zeitlichen Reihenfolge der zwei Beobachtungslaserpulse (B) und des Anregungspulses (A) zur Ermittlung eines Deformationsprofils mit dem Zeitindex Δt;
- Fig. 2: die Darstellung von vier Deformationsprofilen aus Messdaten an einem Silikon-Phantom zu vier verschiedenen Zeitindizes.

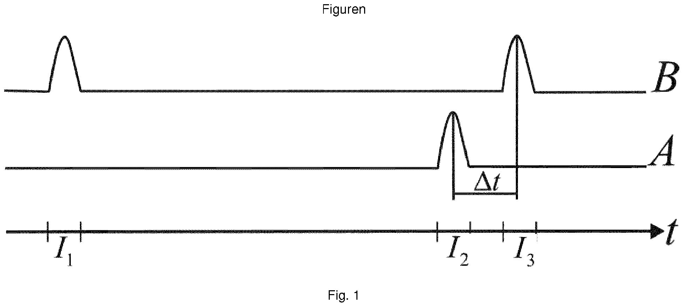

Zur Ermittlung eines einzelnen Deformationsprofils der Messfläche werden drei Lichtpulse wie in Fig. 1 skizziert eingestrahlt. Die Einstrahlung der drei Lichtpulse erfolgt in drei vorzugsweise nichtüberlappenden Zeitintervallen (*I*₁, *I*₂, *I*₃) nacheinander, wobei
... in einem ersten Zeitintervall (*I*₁) die Messfläche mit Beobachtungslicht aus einer Laserlichtquelle beleuchtet wird;
... in einem zweiten Zeitintervall (*I*₂) die Probe mit Anregungslicht beleuchtet wird, das in das Innere der Probe eintreten und von den spektral adressierbaren Zielstrukturen absorbiert werden kann;
... in einem dritten Zeitintervall (*I*₃) eine weitere Beleuchtung der Messfläche mit Beobachtungslicht aus der Laserlichtquelle erfolgt.

Der Mittenabstand zwischen dem zweiten und dem dritten Zeitintervall ist der Zeitabstand Δt, zu dem ein Deformationsprofil bestimmt wird. Das Deformationsprofil wird wie im Stand der Technik beschrieben aus den detektierten Specklemustern berechnet, die zu den Beobachtungslaserpulsen des ersten und des dritten Zeitintervalls von einer elektronischen Kamera erfasst werden. Insbesondere repräsentiert das Deformationsprofil dann die Änderung des Deformationszustandes der Probenoberfläche zwischen dem ersten Zeitintervall (vor Eintreffen des Anregungspulses) und dem dritten Zeitintervall (Δt nach dem Eintreffen des Anregungspulses).

Der Beobachtungslaserpuls, der im ersten Zeitintervall auf die Messfläche eingestrahlt wird, wird als "vorlaufender" Beobachtungslaserpuls bezeichnet. Ihm folgt zeitlich der Anregungspuls, dem sich wiederum im dritten Zeitintervall der "nachlaufende" Beobachtungslaserpuls anschließt.

Der Anregungspuls kann ein Laserlichtpuls sein. Er kann auch aus einer anderen Lichtquelle stammen, die dazu ausgebildet ist, zu vorgegebenen Zeitpunkten Lichtpulse auszusenden, beispielsweise eine Blitzlampe. Die Pulsdauer des Anregungspulses sollte zur Maximierung der Druckamplitude vorzugsweise kleiner als die akustische Transitzeit des Absorbers (üblich definiert als Durchmesser des Absorbers dividiert durch die Schallgeschwindigkeit im Absorber) sein und in der Größenordnung Nanosekunden liegen. Vorzugsweise beträgt die Pulsdauer weniger als 100 ns, besonders bevorzugt weniger als 10 ns.

Erfindungsgemäß soll das zweite Zeitintervall, in dem die Probe mit Anregungslicht beleuchtet wird, zwischen dem ersten und dritten Zeitintervall hinsichtlich seines Zeitabstandes zum dritten Zeitintervall variierbar sein. Dies ist etwa zu erreichen, indem das Aussenden des Anregungspulses durch eine elektronische Ansteuerung ausgelöst wird, die durch eine Steuereinheit (Triggerbox) kontrolliert wird. Die Steuereinheit kann dabei den Zeittakt einer inneren Uhr verwenden. Sie kann auch Signale erhalten, die das Aussenden eines Beobachtungslaserpulses anzeigen, und diese Signale zur zeitlichen Synchronisation verwenden. Solche Signale können vom Beobachtungslaser oder von seiner Ansteuerung oder auch von einer Photodiode herrühren, auf die ein Anteil des Beobachtungslaserlichts gelenkt wird.

Es liegt auch im Rahmen der Erfindung, den Zeitabstand des ersten und dritten Zeitintervalls, also des vorlaufenden und nachlaufenden Beobachtungslaserpulses, während der N-maligen Wiederholung der Abfolge der drei Lichtpulse zu variieren. Auch dann kann jeder Abfolge der Lichtpulse ein vorbestimmter Zeitabstand Δt zwischen dem Anregungspuls und dem nachlaufenden Beobachtungslaserpuls zugewiesen werden, ggf. unter Nutzung der vorgenannten Synchronisation.

Zeichnet die elektronische Kamera die Specklemuster der Beobachtungslaserpulse über eine vorbestimmte Anzahl N von Wiederholungen der Pulsfolge auf, so kann aus den Messdaten eine Sequenz von Deformationsprofilen berechnet werden. Jedem Deformationsprofil der Sequenz wird ein anderer Zeitabstand Δt zugeordnet.

Es ist eine bevorzugte Ausgestaltung der Erfindung, den vorbestimmten Zeitabstand Δt monoton zu variieren, d.h. entweder bei jeder Wiederholung zu steigern oder zu verringern. In diesem Fall soll vom Inkrementieren des Zeitabstandes gesprochen werden, wobei das Inkrement dem Betrage nach variabel sein kann. Das Inkrement weist aber keine Vorzeichenwechsel auf. Es soll dem Betrage nach in der Größenordnung Nanosekunden liegen und bevorzugt 1-100 ns, besonders bevorzugt 10-50 ns betragen.

Eine besonders vorteilhafte - weil technisch einfache - Realisation der zeitlichen Kontrolle der drei Pulse gelingt dadurch, dass man einen repetierenden, gepulsten Beobachtungslaser mit fester Pulsfrequenz PB und eine ebenfalls repetierende, gepulste Anregungslichtquelle mit fester Pulsfrequenz PA benutzt, wobei PA geringfügig größer oder kleiner als PB/2 eingerichtet ist, d.h. 0 < |PA-PB/2| << |PA|. Die Wahl der Differenz von PA und PB/2 legt direkt das - nunmehr konstante - Inkrement fest. Es werden dann in einer einzelnen Abfolge von drei Lichtpulsen je zwei Beobachtungslaserpulse und ein Anregungspuls auf die Probe eingestrahlt, wobei der Anregungspuls bei jeder weiteren Wiederholung der Pulsabfolge etwas früher oder später eintrifft bezogen auf den nachlaufenden Beobachtungslaserpuls.

Die Gesamtheit aller bestimmten Deformationen bilden ein Datenarray, welches eine Funktion von Ort - indiziert durch Pixelkoordinaten der Kamera - und Zeit - indiziert durch einen Zeitindex, der den im Zuge der Sequenz inkrementierten Werten von Δt entspricht - repräsentiert. Dieser in Form elektronischer Daten vorliegende "Film" der Messflächendeformation ist eine geeignete Eingabegröße für eine Rekonstruktionsroutine zur Lokalisierung der Quellen der Druckwellen.

Eine Sequenz aus 100 Deformationsprofilen, deren Zeitabstände beispielsweise um jeweils 50 ns inkrementiert sind, deckt die ersten 5 µs nach dem Eintreffen des Anregungspulses ab. Bei einer vorbekannten Schallgeschwindigkeit der Probe von beispielsweise 1 km/s können dann nur Druckwellen von Zielstrukturen erfasst werden, die weniger als 5 mm unter der Oberfläche liegen, auch wenn das Anregungslicht tiefer eindringen kann. Die Datenaufzeichnung zur Errechnung eines solchen Filmes erfordert - typischerweise, aber nicht zwingend (s.u.) - das elektronische Abspeichern von 200 Ablichtungen der Probe mit dem Beobachtungslaser.

Die auf der elektronischen Kamera erfassten Messwerte mit den jeweils zugeordneten Pixelkoordinaten sowie dem jeweiligen Zeitabstand Δt zwischen dem zweiten und dritten Zeitintervall werden aufgezeichnet, vorzugsweise in einem elektronischen Speicher zur rechnergestützten Nachbearbeitung gespeichert und/oder auf ein nicht-flüchtiges elektronisches Speichermedium geschrieben. Die Nachbearbeitung zur Berechnung der Specklephasen und der zeitaufgelösten Deformation der Probenoberfläche kann mehrere Minuten benötigen, doch ist diese Nachbearbeitungsdauer nicht von Belang.

Entscheidend ist vielmehr die Dauer der Datenerfassung (i. F.: Akquisitionszeit), die mit der Erfindung nun unter zehn Sekunden, bevorzugt unter einer Sekunde, besonders bevorzugt sogar unter 200 ms, bleiben kann. Mit einer kommerziell erhältlichen Kamera mit Bildrate von z.B. 2000 fps (frames per second) können binnen 200 ms bis zu 400 Ablichtungen bzw. 200 Deformationsprofile erfasst werden, die dann eine sehr kurze Zeitspanne nach der Absorption eines Anregungspulses, z.B. bis zu 10 µs, repräsentieren.

Im soweit beschriebenen Verfahren wird jeder Beleuchtungspuls während einer Kamerabelichtung (d.h. in einem Zeitintervall mit der Länge der Verschlusszeit der Kamera) separat aufgezeichnet.

Durch Veränderung der Repetitionsraten der Strahlungsquellen und ggf. Variieren der Kamerabildrate kann das Akquisitionsverfahren beschleunigt oder verzögert werden. Die Wahl der Akquisitionszeit (oder der Samplingrate, wenn die Anzahl der Wiederholungen der Pulsfolge als N vorgegeben ist) muss unter Beachtung der zu erwartenden Eigenbewegung der Probe und des durch die Anregungsstrahlung erfolgenden Energieeintrags in die Probe pro Zeiteinheit geschehen. Bei sich schneller bewegenden Proben sollte die Samplingrate höher sein als bei ruhenden oder sich nur langsam bewegenden Proben. Hierdurch gelangt jedoch auch mehr Energie pro Zeiteinheit in die Probe, was bei gegebener Anregungsenergie in einer thermischen Schädigung resultieren kann.

Eine Verkürzung der gesamten Akquisitionszeit ist möglich, wenn mehrere Einzelpulsbelichtungen, die während nur einer Kamerabelichtung zusammen aufgezeichnet werden, im Nachhinein rechnerisch separierbar sind. Dies gelingt, wenn die Existenz des Zeitabstandes der Laserpulse in die Kameraaufzeichnung hineincodiert werden kann, wie die folgende vorteilhafte Ausgestaltung aufzeigt:

In die von Pedrini et al. beschriebene Ausführungsform der DP-ESPI mit einem ersten Fringemuster auf dem Sensor wird vorzugsweise ein zweites Fringemuster auf dem Sensor eingeführt, das orthogonal zum ersten verläuft. Dies ist beispielsweise dadurch möglich, dass das Laserlicht vor Erreichen der Probe in einen Probenstrahl und zwei Referenzstrahlen aufgeteilt wird, wobei jeder Referenzstrahl über einen eigenen Referenzarm des Interferometers zur Kamera geführt wird. Die Referenzarme können aus unterschiedlichen Winkeln auf die Kamera einstrahlen, insbesondere jeweils unter einem Winkel gegenüber der optischen Achse der Kamera derart, dass zueinander senkrecht verlaufende Phasenrampen des Referenzlichts entlang der horizontalen und der vertikalen Pixelachse gebildet werden. Bei Interferenz mit dem entlang der optischen Achse einfallenden Probenstrahl entstehen dann zueinander orthogonale Fringemuster.

Wenn man weiterhin dafür Sorge trägt, das beim Erfassen des Interferogramms des vorlaufenden Beobachtungslaserpulses nur der erste Referenzarm Licht auf die Kamera einstrahlt und für den nachlaufenden Puls nur der zweite, dann entsteht eine Kameraaufzeichnung mit komplexer Fringestruktur, die hiernach zeilen- und/oder spaltenweise ausgewertet werden kann, um die Phasenprofile für beide Beleuchtungen zu ermitteln. Somit ist letztlich ein Deformationsprofil mit nur einer Kamerabelichtung bestimmbar.

Eine alternative Codierung zweier Beobachtungslaserpulse in einer einzelnen Kameraaufzeichnung kann in der Nutzung der Polarisation des Beobachtungslichts bestehen. Mit einer Maskierung auf den Kamerapixeln, die jeden Pixel für nur eine Polarisationsrichtung sensitiv macht, und einer beispielsweise "schachbrettartigen" Maskenstruktur können beide Beobachtungslaserpulse durch einen schnell schaltenden Polarisator geschickt und so in je einen von zwei orthogonalen Polarisationszuständen überführt werden. Sofern die Probe die Polarisation bei der Streuung weitgehend erhält, zeichnet die Kamera das Interferogramm des ersten Pulses auf anderen Pixeln auf als das des zweiten Pulses. Beide sind dann im gemeinsamen Bild der Kamera direkt durch die Adressierung der Pixel zu separieren.

Ist eine Bildgebung mit geringerer tomographischer Auflösung gewünscht oder für die Zwecke des Nutzers ausreichend, so kann die zeitliche Abtastung mit geringerer Samplingrate erfolgen, was zu weniger Repetitionen und somit zu weniger eingestrahlter Anregungsenergie pro Zeiteinheit führt. Insbesondere kann es zweckmäßig sein, die Samplingrate zu verkleinern, wenn man in erster Linie an tief unter der Probenoberfläche lokalisierten Zielstrukturen interessiert ist. Die von dort ausgehenden Druckwellen weisen ein Schallfrequenzspektrum auf und durchlaufen aufgrund frequenzabhängiger Dämpfung in vielen Probenmaterialien während des Propagierens zur Probenoberfläche einen Tiefpass. Die Deformationen der Probenoberfläche durch weit laufende Druckwellen sind somit auch mit verringerter Zeitauflösung gut erfassbar, was die Probe ebenso durch weniger Anregungspulse pro Zeiteinheit geringer belastet.

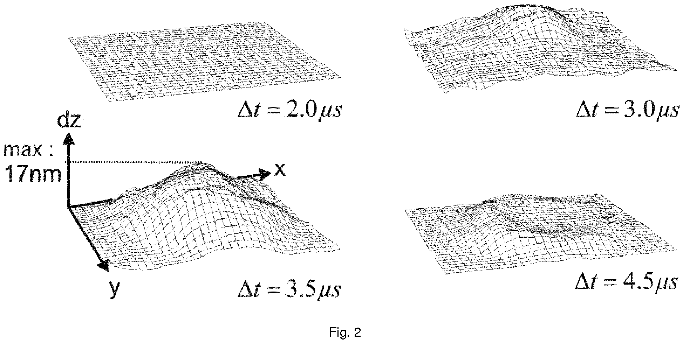

In Fig. 2 sind beispielhaft erfindungsgemäß bestimmte Deformationsprofile an einem Probenphantom zu sehen. Der zugrundeliegende Messaufbau entspricht im Prinzip dem von Pedrini et al., in den zusätzlich ein gepulster Anregungslaser integriert wird.

Konkret wird λ = 1064 nm als Wellenlänge des Anregungslasers mit einer Pulsdauer von 20 ns und einer Pulsenergie von etwa 20 mJ gewählt. Als Beobachtungslaserlicht wird die Wellenlänge 532 nm mit der Pulsdauer 1 ns und der Kohärenzlänge 3 mm benutzt. Etwa ein Neuntel der Intensität des Beobachtungslasers wird vor dem Phantom als Referenzstrahl ausgespiegelt und auf die Kamera geführt. Die Kamera weist die nachfolgenden Kenndaten auf: Progressive Scan 1" CCD-Sensor mit 1600 x 1200 Pixeln, Pixelgröße 7,4 x 7,4 µm², maximale Bildrate 35 fps. Hierbei ist anzumerken, dass diese geringe Bildrate natürlich nur für ein ruhendes Phantom ausreicht. Leider stand zum Zeitpunkt der Messung noch keine schnellere Kamera zur Verfügung. Der Fachmann sieht aber nun ohne weiteres ein, dass sich die insgesamt erforderliche Messdauer entsprechend der Erhöhung der Bildrate verkürzen wird.

Das Probenphantom ist ein Würfel von etwa einem Zentimeter Kantenlänge. Das Matrixmaterial ist hierbei für sichtbares Licht transparentes Silikon, in dessen Innern sich ein kugelförmiger Lichtabsorber (Durchmesser etwa 2 mm) aus schwarzem Silikon mit hoher optischer Absorption befindet. Zusätzlich ist die Seite des Würfels, welche mit dem Beleuchtungslicht bestrahlt wird, mit einer dünnen Schicht weißem Silikon überzogen. Weißes Silikon verfügt über einen hohen optischen Streukoeffizienten. Dadurch wird die Rückstreuung erhöht und ein tiefes Eindringen der Beleuchtungsstrahlung in das in diesem Modell transparente Matrixmaterial verhindert.

Proben, die mit PAT untersucht werden, besitzen oft nicht ideale Eigenschaften für eine DP-ESPI-Messung. Da das Probenmaterial für das Anregungslicht eine hohe Eindringtiefe aufweisen soll, ist es nicht immer möglich, ein Beobachtunglaserlicht bereitzustellen, das ausschließlich an der Probenoberfläche (der Messfläche) gestreut wird. Vielmehr ist mit einem begrenzten Eindringen in eine - oft nur einige Mikrometer dicke - Schicht unter der Messfläche zu rechnen, aus der dann rückgestreutes Licht auf den Detektor gelangen kann. Es kann für die DP-ESPI-Messung vorteilhaft sein, die Messfläche mit einer Streupartikel enthaltenden Schicht zu überziehen, die vornehmlich die Streuung des Beobachtungslaserlichts fördert. Hierfür kommen vorzugsweise keramische Partikel, beispielsweise ein Titanoxid, in Betracht.

Zur Deformationsmessung am würfelförmigen Silikon-Phantom werden Anregungs- und Beobachtungslicht auf verschiedene Würfelseiten eingestrahlt. Die Kamera ist der mit Beobachtungslicht beleuchteten Würfelseite zugewandt und bildet diese ab. In eine der benachbarten Würfelseiten, die senkrecht zur Beobachtungsseite orientiert ist, wird das Anregungslicht eingestrahlt, das in das transparente Matrixmaterial zum Absorber eindringt und die Druckwellenemission initiiert. Messergebnisse der Deformation des Probenphantoms zu ausgewählten Zeitpunkten 2 µs, 3 µs, 3.5 µs und 4.5 µs nach der Einstrahlung von Anregungslicht sind in Fig. 2 dargestellt. Die Absorberkugel im Phantom ist in etwa 3 mm Abstand zur der Kamera zugewandten Würfeloberfläche angeordnet. Aus der Schallgeschwindigkeit in Silikon (ca. 1 km/s) ergibt sich der zu erwartende Zeitpunkt der Oszillation der Oberfläche zu etwa 3 µs nach Anregung. Im ersten Teil der Figur nach 2 µs ist eine ebene Fläche zu sehen, demnach gibt es zu diesem Zeitpunkt keine erkennbare Deformation. Nach den erwarteten 3 µs bildet sich eine kugelförmige Wölbung der Oberfläche aus, welche nach 3.5 µs stärker wird und 4.5 µs nach Anregung zu kollabieren scheint. Die Deformationen werden durch die Erfindung auch quantitativ bestimmt. Die maximale detektierte lokale Auslenkung beträgt 17 nm.

Zusammenfassend adaptiert die Erfindung die an sich bekannte Doppelpuls ESPI zur Nutzung in der Photoakustischen Tomographie. Dies gelingt durch die Hinzunahme eines dritten Lichtpulses, der der Anregung der Probe zu Druckoszillationen dient und als solcher nicht direkt von der Kamera detektiert wird, dessen vorbekannter Zeitpunkt des Aussendens zwischen den beiden Beobachtungslaserpulsen jedoch einen Zeitindex definiert, der einem bestimmbaren Deformationsprofil sachgerecht zuzuordnen ist. Durch Wiederholen der Pulsabfolgen unter Veränderung des Zeitabstandes zwischen Anregungspuls und nachlaufendem Beobachtungslaserpuls kann so eine Sequenz von Deformationsprofilen bestimmt werden, die die wahre Deformation der Messfläche nach dem Applizieren eines einzelnen Anregungspulses approximiert.

Die Erfindung erlaubt zudem technisch die Erfassung aller nötigen Messdaten für Flächen der Größenordnung Quadratzentimeter innerhalb weniger Sekunden oder sogar innerhalb von 200 Millisekunden. Die erfassten Daten sind hiernach noch zu Prozessieren und einer PAT-Rekonstruktion zuzuführen, um auf die Lage der Zielstrukturen zu schließen und/oder diese darzustellen. Doch die schnell abgeschlossene Datenerfassung vermeidet Störungen und Messartefakte, vor allem wenn sich diese aus Eigenbewegungen oder inneren Bewegungen der Probe ergeben würden.

Die Erfindung kann insofern als "Tripelpuls PAT" bezeichnet werden. Sie stellt eine sehr zweckmäßige Weiterbildung des bekannten PAT-Verfahrens dar, indem sie die Nutzung moderner Hochgeschwindigkeitskameras in das PAT-Verfahren einbindet und so das aktuell vorhandene Problem der zu langsamen Datenakquisition fundamental neu löst.

## Patentansprüche

1. Verfahren zur Photoakustischen Tomographie einer Probe, wobei an einer Messfläche auf der Probe Deformationen als Funktion von Ort und Zeit gemessen werden, die sich durch Absorption einer gepulsten Anregungsstrahlung an wenigstens einer spektral adressierbaren Zielstruktur im Probeninneren unter Emission thermomechanischer Druckwellen in Richtung der Messfläche ergeben, und wobei die gemessenen Deformationen einem Rekonstruktionsverfahren zur Bestimmung der Lage der Zielstruktur im Probeninneren zugeführt werden, **dadurch gekennzeichnet, dass**
a. zu einem Anregungspuls (I₂) ein vorlaufender (I₁) und ein nachlaufender (I₃) Beobachtungslaserpuls auf die Messfläche eingestrahlt werden, wobei
b. ein zweidimensionales Deformationsprofil der Messfläche aus den von der Messfläche gestreuten Beobachtungslaserpulsen (I_{1,3}) mittels der Methode der Doppelpuls Elektronischen Speckle-Interferometrie (ESPI) bestimmt wird und
c. der Zeitabstand zwischen dem Anregungspuls (I₂) und dem nachlaufenden Beobachtungslaserpuls (I₃) dem zuvor bestimmten Deformationsprofil als Zeitindex zugeordnet wird und
d. eine vorbestimmte Zahl von Wiederholungen der Schritte a bis c unter Variieren wenigstens des Zeitabstandes zwischen Anregungspuls (I₂) und nachlaufendem Beobachtungslaserpuls (I₃) erfolgen.

2. Verfahren nach Anspruch 1, **dadurch gekennzeichnet, dass** die Anregungspulse (I₂) Pulsdauern von höchstens 100 Nanosekunden, bevorzugt höchstens 10 Nanosekunden, aufweisen.

3. Verfahren nach einem der vorangehenden Ansprüche, **dadurch gekennzeichnet, dass** die Wiederholungen der Schritte a bis c unter Variieren des Zeitabstandes zwischen vorlaufendem (I₁) und nachlaufendem Beobachtungslaserpuls (I₃) erfolgen.

4. Verfahren nach einem der vorangehenden Ansprüche, **dadurch gekennzeichnet, dass** die Wiederholungen der Schritte a bis c unter Inkrementieren des Zeitabstandes zwischen Anregungspuls (I₂) und nachlaufendem Beobachtungslaserpuls (I₃) erfolgen.

5. Verfahren nach einem der vorangehenden Ansprüche, **dadurch gekennzeichnet, dass** Anregungspulse (I₂) mit der Pulsrate PA und Beobachtungslaserpulse (I_{1,3}) mit der Pulsrate PB eingestrahlt werden, wobei die Bedingung 0 < |PA-PB/2| << |PA| erfüllt ist.

6. Verfahren nach einem der vorangehenden Ansprüche, **dadurch gekennzeichnet, dass** das Inkrement des Zeitabstandes zwischen Anregungspuls (I₂) und nachlaufendem Beobachtungslaserpuls (I₃) dem Betrage nach aus dem Intervall 1-100 Nanosekunden, bevorzugt 10-50 Nanosekunden, gewählt wird.

7. Verfahren nach einem der vorangehenden Ansprüche, **dadurch gekennzeichnet, dass** das Streuvermögen der Messfläche für das Beobachtungslaserlicht (I_{1,3}) durch Aufbringen von Partikeln gesteigert wird.

8. Verfahren nach Anspruch 7, **dadurch gekennzeichnet, dass** keramische Partikel, vorzugsweise Titanoxid-Partikel, auf die Messfläche aufgebracht werden.

9. Verfahren nach einem der vorangehenden Ansprüche, **dadurch gekennzeichnet, dass** die gesamte Datenerfassung in weniger als zehn Sekunden, bevorzugt in weniger als einer Sekunde, besonders bevorzugt in weniger als 200 Millisekunden, durchgeführt wird.

## Claims

1. Method for photoacoustic tomography of a sample, whereby deformations on the measurement surface of the sample being measured as a function of location and time, the deformations resulting from the absorption of a pulsed excitation radiation on at least one spectrally addressable target structure within the interior of the sample while emitting thermomechanical pressure waves in the direction of the measurement surface, and whereby the measured deformations being fed to a reconstruction method for determining the position of the target structure in the interior of the sample, **characterized in that**
a. an excitation pulse (I₂) and a leading (I₁) and a trailing (I₂) observation laser pulse relative to then excitation pulse are irradiated onto the measurement surface,
b. a two-dimensional deformation profile of the measurement surface being determined from the observation laser pulses (I₁, I₃) scattered by means of a method of double-pulse electronic speckle interferometry (ESPI), and
c. the temporal distance between the excitation pulse (I₂) and the trailing observation laser pulse (I₃) being is assigned to the predetermined two-dimensional deformation profile as a time index, and
d. a predetermined number of repetitions of steps a to c taking take place by varying at least the temporal distance between the excitation pulse (I₂) and the trailing observation laser pulse (I₃).

2. Method according to Claim 1, **characterized in that** the excitation pulses (I₂) exhibits a pulse duration of at most 100 nanoseconds, preferably at max 10 nanoseconds.

3. The method according to one of the preceding claims, **characterized in that** the repetitions of the steps a to c take place while varying the temporal distance between the leading (I₁) and the trailing observation (I₃) laser pulses.

4. Method according to one of the preceding claims, **characterized in that** the repetitions of steps a to c take place while incrementing the temporal distance between the excitation pulse (I₂) and the trailing observation laser pulse (I₃).

5. Method according to one of the preceding claims, **characterized in that** the excitation pulses (I₂) having the a pulse rate PA, and observation laser pulses (I₁, I₃) having a pulse rate PB, are irradiated, whereby the condition 0 < |PA-PB/2| << |PA| is fulfilled.

6. Method according to one of the preceding claims, **characterized in that** the increment of the temporal distance between the excitation pulse (I₂) and the trailing observation pulse (I₂) is selected from the interval 1-100 nanoseconds, preferably 10-50 nanoseconds.

7. Method according to one of the preceding claims, **characterized in that** the scattering power from the measurement surface for the observation laser light (I₁, I₃) is enhanced by applying particles.

8. Method according to claim 7, **characterized in that** ceramic particles, preferably titanium-oxide particles, are applied to the measurement surface.

9. Method according to according to one of the preceding claims, **characterized in that** the entire data acquisition is carried out in less than ten seconds, preferably in less than one second, more preferably in less than 200 milliseconds.

## Revendications

1. Procédé de tomographie photoacoustique d'un échantillon, sachant que des déformations sont mesurées sur une surface de mesure sur l'échantillon en fonction du lieu et du temps, qui résultent par absorption d'un rayonnement d'excitation pulsé sur au moins une structure cible spectralement adressable à l'intérieur de l'échantillon en émettant des ondes de pression thermo-mécaniques en direction de la surface de mesure et sachant que les déformations mesurées sont dirigées vers un procédé de reconstruction pour déterminer la position de la structure cible à l'intérieur de l'échantillon, **caractérisé en ce que**
a. pour une impulsion d'excitation (I₂), une impulsion laser d'observation antérieure (I₁) et une ultérieure (I₃) sont irradiées sur la surface de mesure pour une impulsion d'excitation (I₂), sachant que
b. un profil de déformation bidimensionnelle de la surface de mesure est déterminé à partir des impulsions laser d'observation (I₁, I₃) dispersées par la surface de mesure au moyen de la méthode de l'Interférométrie de Speckle électronique (ISPE) à impulsion double, et
c. l'intervalle de temps entre l'impulsion d'excitation (I₂) et l'impulsion laser d'observation ultérieure (I₃) est attribué au profil de déformation préalablement déterminé en tant qu'indice temporel, et
d. un nombre préalablement déterminé de répétitions des étapes a à c ont lieu en modifiant au moins l'intervalle de temps entre l'impulsion d'excitation (I₂) et l'impulsion laser d'observation ultérieure (I₃).

2. Procédé selon la revendication 1, **caractérisé en ce que** les impulsions d'excitation (I₂) comportent des durées d'impulsion d'un maximum de 100 nanosecondes, de préférence au maximum de 10 nanosecondes.

3. Procédé selon l'une quelconque des revendications précédentes, **caractérisé en ce que** les répétitions des étapes a à c ont lieu en modifiant l'intervalle de temps entre l'impulsion laser d'observation antérieure (I₁) et l'impulsion laser d'observation ultérieure (I₃).

4. Procédé selon l'une quelconque des revendications précédentes, **caractérisé en ce que** les répétitions des étapes a à c ont lieu en augmentant l'intervalle de temps entre l'impulsion d'excitation (I₂) et l'impulsion laser d'observation ultérieure (I₃).

5. Procédé selon l'une quelconque des revendications précédentes, **caractérisé en ce que** les impulsions d'excitation (I₂) sont irradiées avec la vitesse d'impulsion PA et les impulsions laser d'observation (I₁, I₃) avec la vitesse d'impulsion PB, sachant que la condition 0<|PA-PB/2|<<|PA| est remplie.

6. Procédé selon l'une quelconque des revendications précédentes, **caractérisé en ce que** l'augmentation de l'intervalle de temps entre l'impulsion d'excitation (I₂) et l'impulsion laser d'observation ultérieure (I₃) est choisie en fonction du montant à partir de l'intervalle de 1-100 nanosecondes, de préférence de 10-50 nanosecondes.

7. Procédé selon l'une quelconque des revendications précédentes, **caractérisé en ce que** le pouvoir de dispersion de la surface de mesure est réhaussé pour la lumière laser d'observation (I₁, I₃) par application de particules.

8. Procédé selon la revendication 7, **caractérisé en ce que** des particules céramiques, de préférence des particules d'oxyde de titane, sont appliquées à la surface de mesure.

9. Procédé selon l'une quelconque des revendications précédentes, **caractérisé en ce que** toute la saisie des données est exécutée en moins de dix secondes, de préférence en moins d'une seconde, en particulier de préférence en moins de 200 millisecondes.
